# EUROPEAN PATENT APPLICATION

(11) **EP 4 667 066 A1**
(43) Date of publication of application: **24.12.2025**
(21) Application number: 24712879.6
(22) Date of filing: 16.02.2024
(51) Int. Cl.: A63B 22/18, A63B 26/00, A61B 5/11, A61H 1/00, A61H 1/02, A63B 22/00

(54) **DEVICE FOR DIAGNOSING AND REHABILITATING BALANCE WHEN STANDING AND WALKING, AND OPERATING METHOD OF AN INSTALLATION FORMED BY TWO OF SAID DEVICES**

(30) Priority: 16.02.2023 ES 202330119
(71) Applicant: Universidad del País Vasco/Euskal Herriko Unibertsitatea, 48940 Leioa, Vizcaya (ES); ADMINISTRACIÓN GENERAL DE LA COMUNIDAD AUTÓNOMA DE EUSKADI, 01010 Vitoria-Gasteiz (Álava) (ES)
(72) Inventor: PINTO, Charles, 48940 Leioa (Vizcaya) (ES); HERRERO VILLALIBRE, Saioa, 48940 Leioa (Vizcaya) (ES); CORRAL SAIZ, Javier, 48940 Leioa (Vizcaya) (ES); DIEZ SÁNCHEZ, Mikel, 48940 Leioa (Vizcaya) (ES); MACHO MIER, Erik, 48940 Leioa (Vizcaya) (ES); CAMPA GÓMEZ, Francisco Javier, 48940 Leioa (Vizcaya) (ES); DIEGO MARTÍN, Paul, 48940 Leioa (Vizcaya) (ES); IGNAZIO TEJADA, Pedro, 01010 Vitoria-Gasteiz (Álava) (ES); GARCÍA ORTIZ, María Mar, 01010 Vitoria-Gasteiz (Álava) (ES); LÓPEZ MARTÍNEZ, Héctor, 01010 Vitoria-Gasteiz (Álava) (ES)
(74) Representative: Balder IP Law, S.L.
(86) International application number: PCT/ES2024/070088
(87) International publication number: WO 2024/170814

(57) **Abstract**

The invention discloses a device (1) for diagnosing and rehabilitating balance that comprises a platform (2) supported by a kinematic mechanism (3). The kinematic mechanism (3) comprises four kinematic chains (31, 32, 33, 34). The first, second and third kinematic chains (31, 32, 33) are the same, each of them comprising a proximal rod (BP31, BP32, BP33) attached by respective passive spherical couples (PEI31, PEI32, PEI33) to a distal rod (BD31, BD32, BD33), and each being attached to a fixed base (B) by actuated proximal rotational couples (PRPA31, PRPA32, PRPA33) and to the platform (2) through passive distal spherical couples (PED31, PED32, PED33). The fourth kinematic chain (34) is attached to the fixed base (B) by an actuated proximal rotational couple (PRPA34) and to the platform (2) by a universal joint (JU34).

## Description

### OBJECT OF THE INVENTION

The present invention belongs to the field of medicine, and more particularly to diagnosing and rehabilitating, both in standing and walking, of patients with balance deficiencies.

A first object of the invention is a device designed to perform programmed movements to diagnose and rehabilitate patients who have balance deficiencies both when standing and walking.

A second object of the present invention is an operating method of an installation formed by two devices as described above.

### BACKGROUND OF THE INVENTION

Currently, devices are known that are designed for diagnosing or rehabilitating patients with balance deficiencies. These devices are normally based on one or two platforms configured to perform a series of programmed rotation and/or translation movements. To perform a balance test, a patient is positioned standing on the platform of the device and is asked to attempt to remain standing regardless of the movements of the platform. The patient's balance status is evaluated depending on the patient's ability to achieve this, the features of the supports, as well as the possible need to hold on during the test. Alternatively, the device can be used to rehabilitate patients with balance deficiencies by generating programs for therapeutic purposes.

In this context, it is important to note that this type of device must perform a sufficiently wide variety of rotation and/or translation movements. This is important because it increases its ability to implement movement sequences with different degrees of difficulty, allowing the design of diagnostic and rehabilitation programs with complex characteristics adapted to each particular case.

The state of the art comprises installations capable of generating certain movements in a platform to achieve different objectives. For example, document US3645011 describes one of these installations wherein, by means of a front hydraulic cylinder and two rear hydraulic cylinders, said platform can be rotated according to both an anteroposterior movement and a lateral movement, the resulting rotation being a composition of said anteroposterior and lateral rotations. A booth is mounted on the platform in which a person can be located and perceive the movement of the platform as if it were the movement of an aeroplane they are piloting.

There are also installations that generate movement for therapeutic purposes. Document US6162189A describes a device of this type that has two mobile platforms attached to two fixed platforms by means of pneumatic actuators. Each of the mobile platforms can move with respect to the fixed platforms in the directions defined by three perpendicular Cartesian axes and rotate about each of these axes such that it can move with 6 degrees of freedom. Each of these platforms also has a holding element for each of the feet of a person, each holding element being equipped with a force sensor. A controller establishes the movement of the pneumatic actuators in order to define the movement of the mobile platforms. Thus, the forces exerted by the patients' feet in response to the movement of the mobile platforms are measured by means of the force sensors located in the holding elements.

### DESCRIPTION OF THE INVENTION

The applicant of the present invention proposes a new device for diagnosing and rehabilitating balance when standing and walking that uses a novel kinematic mechanism to support the platform. This kinematic mechanism enables a wide variety of rotation, translation, and combined rotation and translation movements to be performed and, at the same time, is relatively easy to control.

The present invention also relates to an operating method of an installation for diagnosing and rehabilitating balance formed by two of said devices.

A series of terms are defined below that will be used throughout this specification:
Proximal/distal: In the natural position of the device of the invention, the kinematic mechanism is in a lower position and fastened to the ground, and the platform rests on said kinematic mechanism and is oriented horizontally. However, other orientations of the device are feasible. For example, the platform could be oriented vertically with the kinematic mechanism fastened to a vertical wall. This configuration could have other uses that are not related to the patient's balance, such as performing upper limb rehabilitation exercises. It would also be possible to rest the device on an inclined plane to increase the difficulty of the exercises. In any case, in order not to restrict the invention to a specific orientation, in this description the terms *"proximal"* and *"distal"* are used to indicate the relative position of the different elements in relation to a base to which the kinematic mechanism is fastened. That is, the *"proximal"* side of a given component is the one that is located closest to the base to which the kinematic mechanism is fastened, and the *"distal"* side of a given component is the one that is located furthest from the base to which the kinematic mechanism is fastened.
XYZ axes: The description of the device of the invention is made with reference to the XYZ directions. The XYZ directions form a Cartesian coordinate system in three dimensions, where the XY plane is parallel to the base on which the kinematic mechanism is fastened. In the natural position of the device, the Z axis would coincide with the vertical direction, while the X and Y directions would be contained in a horizontal plane.
Rotational couple: This term refers to a rotation joint that has a single degree of freedom, that is, a plane joint that only allows rotation in one plane.
Universal joint: This term refers to a joint formed by two axes of rotation perpendicular to each other. This type of joint is also known as a *"cardan joint".*
Spherical couple: This term refers to a rotation joint that has three degrees of freedom, that is, a spherical joint that allows rotation in any direction in space.
Actuated couple/passive couple: The term *"actuated couple"* refers to a joint that is motorised or actuated by suitable means. On the contrary, the term *"passive couple"* refers to a joint that is not motorised or actuated and is therefore a free joint.

### First aspect: device for diagnosing and rehabilitating balance

A first aspect of the present invention relates to a device for diagnosing and rehabilitating balance that comprises a platform supported by a kinematic mechanism configured to impart rotation movements, translation movements, and combined rotation and translation movements to the platform.

In principle, the platform can have any shape as long as it allows at least one foot of the patient to be supported in order to perform the balance exercises. Typically, this is a flat plate that is essentially rectangular in shape and is a size suitable for accommodating a patient's foot. The platform has a proximal face to which the kinematic mechanism is fastened and a distal face on which the patient's foot rests. Furthermore, according to a particularly preferred embodiment of the invention, the distal face of the platform may comprise sensors configured to determine position and reaction force when the patient's foot rests thereon. As mentioned previously in this document, information about the position and reaction force of the patient's foot is relevant to diagnosing the patient's ability to balance.

Furthermore, the kinematic mechanism is the component to which the platform is attached and which imparts rotation and/or translation movements thereto. The kinematic mechanism comprises four kinematic chains: the first, second and third kinematic chains are the same as each other, while a fourth kinematic chain is different from the previous ones.

Each of the first, second and third kinematic chains comprises a proximal rod attached to a distal rod. The proximal rod is closest to the base plane, while the distal rod is closest to the platform. Each proximal rod is contained in a corresponding plane parallel to a Z direction and an X direction. As mentioned above, a Cartesian coordinate axis is used such that the aforementioned X direction and a Y direction perpendicular thereto are contained in a XY base plane that is perpendicular to said Z direction. Each of the proximal rods of the first, second and third kinematic chains is contained in the corresponding plane in all the positions it adopts during the movement of the platform.

Note that, although in this description and in the attached figures the base plane coincides with the fixed base to which the kinematic mechanism is fastened, this is not an essential requirement in the present invention. The fixed base may not be flat, with each kinematic chain fastened at a different distance from the platform. To adapt the device to this configuration it would be enough to appropriately modify the length of the rods. For example, depending on the circumstances, the fixed base could be inclined in relation to the platform, or have different heights.

More particularly, each of the first, second and third kinematic chains comprises a proximal rod attached to a distal rod. Each proximal rod comprises an actuated proximal rotational couple about an axis parallel to the Y direction at a proximal end configured for attachment to the fixed base, and a passive intermediate spherical couple at an intermediate end attached to the distal rod. In turn, each distal rod comprises a passive distal spherical couple at its distal end attached to the proximal face of the platform.

The fourth kinematic chain also comprises a proximal rod attached to a distal rod. Furthermore, the fourth kinematic chain is contained in a plane parallel to the planes containing the proximal rods of the first, second and third kinematic chains. That is, the entire fourth kinematic chain is contained during all the movements of the platform in an XZ plane.

In this case, however, the proximal rod comprises an actuated proximal rotational couple about an axis parallel to the Y direction at a proximal end configured for attachment to the fixed base, and a passive intermediate rotational couple about an axis parallel to the Y direction at an intermediate end attached to the distal rod. In turn, the distal rod comprises, a universal joint having a first axis parallel to the Y direction at a distal end attached to the proximal face of the platform. As is known, the second axis of the universal joint is perpendicular to the first axis, such that it is contained in the plane that contains said fourth kinematic chain.

Thanks to this configuration, the device of the present invention allows a very wide variety of movements of the platform, which can move in the direction of the Z or X axis, rotate about an axis parallel to the X or Y axis, or perform a combination of all of them.

In principle, the planes containing the proximal rod of the first kinematic chain and the proximal rod of the third kinematic chain can be located at different distances from each other. However, in a particularly preferred embodiment of the invention, a first plane containing the proximal rod of the first kinematic chain and a third plane containing the proximal rod of the third kinematic chain coincide. That is, the proximal rods of both kinematic chains are contained in the same XZ plane.

In this case, the planes respectively containing the proximal rod of the second kinematic chain and the proximal rod of the fourth kinematic chain may be located at different distances in relation to the plane containing the proximal rods of the first and third kinematic chains. However, according to another particularly preferred embodiment of the invention, a second plane containing the proximal rod of the second kinematic chain and a fourth plane containing the fourth kinematic chain are equidistant in relation to the plane containing the first and third kinematic chains. Furthermore, said second and fourth planes are located on opposite sides in relation to the plane containing the first and third kinematic chains.

Even more preferably, a distance between the first passive distal spherical couple and an attachment line between the universal joint and the second passive distal spherical couple is the same as a distance between the third passive distal spherical couple and said attachment line between the universal joint and the second passive distal spherical couple. Similarly, a distance between the second passive distal spherical couple and an attachment line between the first passive distal spherical couple and the third passive distal spherical couple is preferably the same as a distance between the universal joint and said attachment line between the first passive distal spherical couple and the third passive distal spherical couple.

The present invention also relates to an installation for diagnosing and rehabilitating balance that comprises two devices as described above, wherein said devices are located next to each other according to the Y direction. This configuration gives the installation great flexibility, such that it can perform a wide variety of movements for diagnosing or rehabilitating the gait of a patient with balance problems. Some of these movements are described below in relation to the operating method of this installation.

In some embodiments, the locations of the first, second, third and fourth kinematic chains of one of the devices of the installation are symmetrical to the locations of, respectively, the third, second, first and fourth kinematic chains of the other device of the installation, the symmetry being with respect to a plane parallel to the X and Z axes located between the two devices of the installation; the distance between the fourth kinematic chain of one of the devices of the installation and the fourth kinematic chain of the other of the devices of the installation being the smallest distance between one of the first, second, third and fourth kinematic chains of one of the devices and one of the first, second, third and fourth kinematic chains of the other of the devices. In this way, it is possible to minimise the separation between the platforms of the two devices of the installation when the platforms rotate with respect to the X axis.

The symmetry between the locations of the kinematic chains does not require the orientation of the kinematic chains to be symmetrical during the movement of the devices. Since each of the devices has the capacity to move independently from the other, the orientation of the mechanisms does not have to have such symmetry, although it could.

To achieve this symmetry it may be enough for:
- the location of the proximal rotational couple of the first kinematic chain of a first device of the installation to be symmetrical with the location of the proximal rotational couple of the third kinematic chain of a second device of the installation;
- the location of the proximal rotational couple of the second kinematic chain of the first device of the installation to be symmetrical with the location of the proximal rotational couple of the second kinematic chain of the second device of the installation;
- the location of the proximal rotational couple of the third kinematic chain of the first device of the installation to be symmetrical with the location of the proximal rotational couple of the first kinematic chain of the second device of the installation; and
- the location of the proximal rotational couple of the fourth kinematic chain of the first device of the installation to be symmetrical with the location of the proximal rotational couple of the fourth kinematic chain of the second device of the installation;
the symmetry being with respect to the plane parallel to the X and Z axes located between the two devices of the installation.

In some embodiments, the installation comprises steps and/or a ramp, the steps and/or the ramp connecting a lower surface according to the Z axis with an upper surface according to the Z axis, the upper surface being on the fixed bases of the devices of the installation, the upper surface being a surface to access the devices. The steps and/or the ramp make it possible to reduce the distance according to the Z axis that the user has to travel to stand on/access the devices.

In some embodiments, the upper surface is below the platforms of the devices of the installation, according to the Z axis. This improves the freedom of movement of the platforms, since by having the upper surface below the platforms, the obstacle that said upper surface represents to the movement of the platforms can be reduced.

In some embodiments, the installation comprises a railing on each side of the steps and/or the ramp. The railings make it easier for the user to get on and off the installation.

In some embodiments, the steps have a width of at least 80 cm, 1 m or 1.2 m, the width being the dimension from the railing on one side of the steps/ramp to the railing on the other side of the steps/ramp. This allows two people to simultaneously get on the upper surface, making it easier for one person (for example, a therapist) to help the other person (for example, a patient) to get on.

In some embodiments, the steps have a width of at least 1.8 m, with one handrail of the handrails separating the right side area of the steps with respect to a central area of the steps and another handrail of the handrails separating the central area of the steps with respect to a left side area of the steps; the central area of the steps having a width of at least 80 cm and each of the right side and left side areas of the steps having a width of at least 50 cm. In this way, support personnel can help, from the right side and left side areas of the steps, a user to get on the upper surface and/or to get off the upper surface.

In some embodiments, the devices are surrounded by a U-shaped structure that the user of the devices can hold, directly or by means of a grip (e.g., a strap) attached to said structure, during use of the devices of the installation. This U-shaped structure improves the stability of the user when using the devices.

In some embodiments, the upper surface at least partially surrounds the platforms of the devices (e.g., the upper surface is U-shaped). In this way, it is easier to help the user of the devices from different angles with respect to the user.

In some embodiments, a structure outside the installation at least partially surrounds the installation and allows the attachment of a safety harness for the user, cameras for capturing the movement of the user of the devices, and light sources to illuminate the installation.

### Second aspect: operating method of an installation comprising two devices

A second aspect of the present invention relates to an operating method of an installation as mentioned above, that is, an installation comprising two devices located next to each other according to the Y direction. This method comprises actuating the respective kinematic mechanisms of the two devices to impart a coordinated movement to the platforms.

The coordinated movements of the platforms can be of many types, although five movements that are particularly useful for diagnosing and rehabilitating a patient's balance are explicitly mentioned herein:
a) In a first particular embodiment, the operating method comprises actuating the kinematic mechanisms of the two devices to impart a rotation movement to the platforms about an axis parallel to the X direction while maintaining the distal faces of both platforms contained in the same plane. The platforms both rotate in synchrony such that they are always next to each other to effectively provide a single flat distal surface throughout the entire movement.
b) In a second particular embodiment, the operating method comprises actuating the kinematic mechanisms of the two devices to impart a rotation movement to the platforms about an axis parallel to the Y direction while maintaining the distal faces of both platforms contained in the same plane. The platforms both rotate in synchrony such that they are always next to each other to effectively provide a single flat distal surface throughout the entire movement.
c) In a third particular embodiment, the operating method comprises actuating the kinematic mechanisms of the two devices to impart alternate rotation movements to the platforms about an axis parallel to the Y direction combined with translation movements according to directions parallel to the X direction and the Z direction that simulate the walking motion. The platforms simulate a walking motion in a manner similar to an elliptical trainer.
d) In a fourth particular embodiment, the operating method comprises actuating the kinematic mechanisms of the two devices to impart an alternate translation movement to the platforms in a direction parallel to the Z direction. That is, the platforms move alternately in the direction of the Z axis, in such a way that while one is moving upwards, the other is moving downwards, both arriving at the same time at the respective change of direction points.
e) In a fifth particular embodiment, the operating method comprises actuating the kinematic mechanisms of the two devices to impart an alternate translation movement to the platforms in a direction parallel to the X direction. That is, the platforms move alternately in the direction of the X axis, in such a way that while one is moving forward, the other is moving backward, both arriving at the same time at the respective change of direction points. During the entire alternating movement, both platforms are next to each other at the same height according to the Z axis.

### BRIEF DESCRIPTION OF THE DRAWINGS

Figure 1 shows a perspective view of an example of a device according to the present invention.
Figure 2 shows another perspective view of the device of the present invention with the translucent platform.
Figure 3 shows another perspective view of the device of the present invention without the platform.
Figures 4a-4c show a first sequence of movements of an installation formed by two devices according to the present invention.
Figures 5a-5c show a second sequence of movements of an installation formed by two devices according to the present invention.
Figures 6a-6c show a third sequence of movements of an installation formed by two devices according to the present invention.
Figures 7a-7c show a fourth sequence of movements of an installation formed by two devices according to the present invention.
Figures 8a-8e show a fifth sequence of movements of an installation formed by two devices according to the present invention.
Figures 9a-9c show different views of an installation formed by two devices according to the present invention.

### PREFERRED EMBODIMENT OF THE INVENTION

A particular example of the present invention is described below with reference to the attached figures.

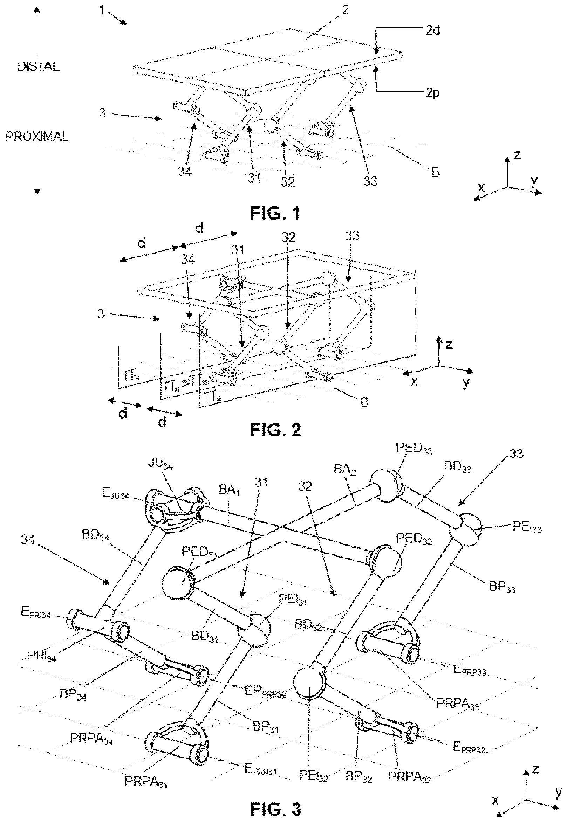

Figure 1 shows an example of a device (1) according to the present invention that comprises a kinematic mechanism (3) that supports a platform (2). As can be seen, the device (1) herein is arranged in its natural position for diagnosing and rehabilitating balance functions when a patient is standing and walking. That is, the kinematic mechanism (3) rests on a fixed base (B) which, in this specific example, consists fundamentally of a horizontal flat surface. In turn, the platform (2) is fixed on said kinematic mechanism (3).

In this context, a three-dimensional Cartesian coordinate system is considered wherein the fixed base (B) coincides with the XY plane and the vertical direction is parallel to the Z axis. That is, the proximal-distal direction coincides with the vertical direction, with the proximal side located below and the distal side above.

The platform (2) is fundamentally a flat rectangular plate that has a proximal (2p) or lower face and a distal (2d) or upper face. During a balance and gait diagnosis and rehabilitation exercise, the patient rests their foot on the distal face (2d) of this platform (2). This distal face (2d) may also have reaction force sensors and position sensors that allow useful information to be obtained to know the patient's movements in detail.

As seen in greater detail in Figures 2 and 3, the kinematic mechanism (3) comprises four kinematic chains (31, 32, 33, 34) that are contained in vertical planes (π₃₁, π₃₂, π₃₃, π₃₄) parallel to the X axis. Next, each of the kinematic chains (31, 32, 33, 34) is defined in greater detail.

A first kinematic chain (31) comprises a first proximal rod (BP₃₁) and a first distal rod (BD₃₁) connected to each other, the proximal rod (BP₃₁) being contained in a first vertical plane (π₃₁) parallel to the X axis. A proximal end of the first proximal rod (BP₃₁) is attached to the fixed base (B) on which the device (1) is arranged through a first actuated proximal rotational couple (PRPA₃₁) of rotation axis (E_{PRP31}) parallel to the Y axis. An intermediate end of the first proximal rod (BP₃₁) is attached to an intermediate end of the first distal rod (BD₃₁) through a first passive intermediate spherical couple (PEI₃₁). A distal end of the first distal rod (BD₃₁) is attached to the proximal face (2p) of the platform (2) through a first passive distal spherical couple (PED₃₁).

A second kinematic chain (32) comprises a second proximal rod (BP₃₂) and a second distal rod (BD₃₂) connected to each other, the proximal rod (BP₃₂) being contained in a second vertical plane (π₃₂) parallel to the X axis. The first plane (π₃₁) and the second plane (π₃₂) are separated by a distance (d). A proximal end of the second proximal rod (BP₃₂) is attached to the fixed base (B) on which the device (1) is arranged through a second actuated proximal rotational couple (PRPA₃₂) of rotation axis (E_{PRP32}) parallel to the Y axis. An intermediate end of the second proximal rod (BP₃₂) is attached to an intermediate end of the second distal rod (BD₃₂) through a second passive intermediate spherical couple (PEI₃₂). A distal end of the second distal rod (BD₃₂) is attached to the proximal face (2p) of the platform (2) through a second passive distal spherical couple (PED₃₂).

A third kinematic chain (33) comprises a third proximal rod (BP₃₃) and a third distal rod (BD₃₃) connected to each other, the proximal rod (BP₃₃) being contained in a third vertical plane (π₃₃) parallel to the X axis. In this specific example, the third plane (π₃₃) coincides with the first plane (π₃₁), such that both are separated by a distance (d) from the second plane (π₃₂). A proximal end of the third proximal rod (BP₃₃) is attached to the fixed base (B) on which the device (1) is arranged through a third actuated proximal rotational couple (PRPA₃₃) of rotation axis (E_{PRP33}) parallel to the Y axis. An intermediate end of the third proximal rod (BP₃₃) is attached to an intermediate end of the third distal rod (BD₃₃) through a third passive intermediate spherical couple (PEI₃₃). A distal end of the third distal rod (BD₃₃) is attached to the proximal face (2p) of the platform (2) through a third passive distal spherical couple (PED₃₃).

A fourth kinematic chain (34) is contained in a fourth vertical plane (π₃₄) parallel to the X axis. The fourth plane (π₃₄) is located at a distance (d) from the first/third plane (π₃₁, π₃₃), on the opposite side thereof in relation to the second plane (π₂). The fourth kinematic chain (34) comprises a fourth proximal rod (BP₃₄) and a fourth distal rod (BD₃₄) connected to each other. A proximal end of the fourth proximal rod (BP₃₄) is attached to the fixed base (B) on which the device (1) is arranged through a fourth actuated proximal rotational couple (PRPA₃₄) of rotation axis (E_{PRP34}) parallel to the Y axis. An intermediate end of the fourth proximal rod (BP₃₄) is attached to an intermediate end of the fourth distal rod (BD₃₄) through a passive intermediate rotational couple (PRI₃₄) of rotation axis (E_{PRI34}) parallel to the Y axis. A distal end of the fourth distal rod (BD₃₄) is attached to the proximal face (2p) of the platform (2) through a universal joint (JU₃₄) which has a first rotation axis (E_{JU34}) parallel to the Y axis. A second rotation axis of the universal joint (JU₃₄) is contained in the fourth plane (π₃₄).

As can be seen, in this specific example, the second plane (π₃₂) and the fourth plane (π₃₄) are separated from the first/third plane (π₃₁, π₃₃) by the same distance (d). Likewise, the third distal spherical couple (PED₃₃) and the first distal spherical couple (PED₃₁) are both separated by an imaginary line joining the universal joint (JU₃₄) and the second distal spherical couple (PED₃₂) also by the same distance (d). Note that, although this symmetrical arrangement of the kinematic chains (31, 32, 33, 34) is advantageous because it facilitates the control of the device (1), it is not an essential requirement in the present invention, such that the kinematic chains (31, 32, 33, 34) could be located in other positions. The only essential requirement in this context is the fact that the proximal rods (BP₃₁, BP₃₂, BP₃₃, BP₃₄) of all kinematic chains (31, 32, 33, 34) are contained in parallel vertical planes.

In this example, the kinematic scheme represented includes some additional elements that are described below in a non-limiting manner. Two additional crossed rods (BA₁, BA₂) have been arranged on the proximal face (2p) of the platform (2) that connect the distal ends of each of the kinematic chains (31, 32, 33, 34) two by two. Specifically, a first additional rod (BA₁) is fastened to the proximal face (2p) of the platform (2) oriented according to the Y axis to connect the universal joint (JU₃₄) and the second distal spherical couple (PED₃₂), while the second additional rod (BA₂) is fastened to the proximal face (2p) of the platform oriented according to the X axis to connect the first distal spherical couple (PED₃₁) and the third distal spherical couple (PED₃₃). In any case, the additional rods (BA₁, BA₂) are not essential in the invention since the fastening of the distal end of each kinematic chain (31, 32, 33, 34) to the proximal face (2p) of the platform (2) will normally be carried out directly using suitable fastening means.

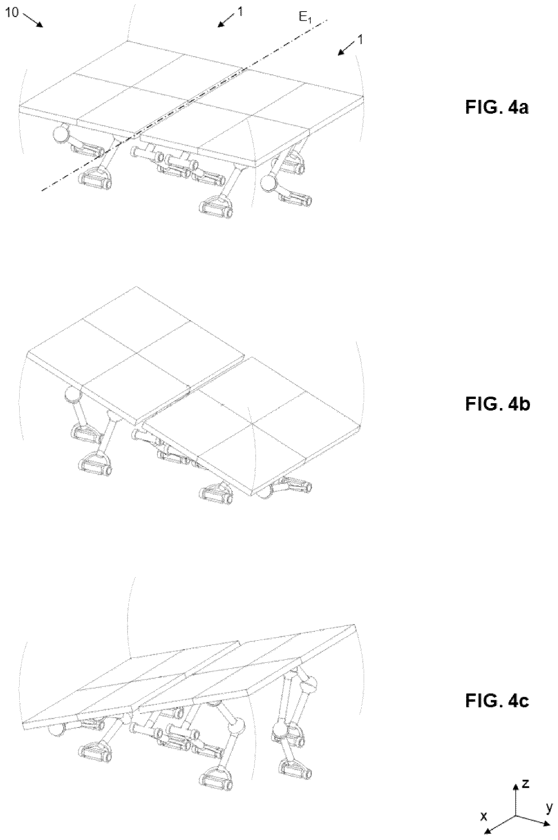

Having fully defined the structure of a device (1) according to the present invention, some examples of movement sequences of an installation (10) for diagnosing and rehabilitating balance formed by two devices (1) of this type are described below. As mentioned previously in this document, the installation (10) is formed by two devices (1) arranged next to each other along a direction parallel to the Y axis. As can be seen in any of Figures 4-8, the two devices (1) are located such that a right edge of the platform (2) of the device (1) located on the left and a left edge of the platform (2) of the device (1) located on the right are parallel and adjacent to each other. For example, taking into account the usual dimensions of this type of device (1), the distance between both platforms (2) could be between 0.5 cm and 5 cm.

Figures 4a-4c show a first example of a sequence of movements that the installation (10) can perform. In this first example, the platforms (2) both rotate in unison in relation to the same axis (E₁) parallel to the X axis. The two platforms (2) are always located next to each other in the same plane to effectively provide a single flat distal surface throughout the entire movement. In this example, as seen in the figures, the rotation axis (E₁) is located in the same plane as the platforms (2) at an intermediate point between the two. Therefore, it is a pure rotation movement. Figure 4a shows a moment of rotation in which both platforms (2) are contained in a horizontal plane. Figure 4b shows a moment in which both platforms (2) are inclined by a maximum angle towards one side of the movement. Figure 4c shows a moment in which both platforms (2) are inclined by the same maximum angle towards the opposite side of the movement.

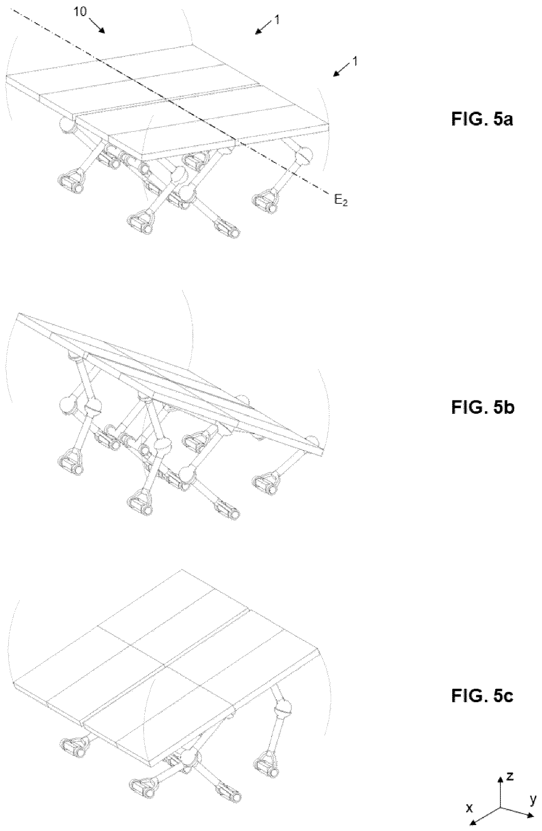

Figures 5a-5c show a second example of a sequence of movements that the installation (10) can perform. In this second example, the platforms (2) both rotate in unison in relation to the same axis (E₂) parallel to the Y axis. The two platforms (2) are always located next to each other in the same plane to effectively provide a single flat distal surface throughout the entire movement. In this example, as seen in the figures, the rotation axis (E₂) passes through a midpoint of the platforms (2) in the direction of the X axis. Therefore, it is another pure rotation movement. Figure 5a shows a moment of rotation in which both platforms (2) are contained in a horizontal plane. Figure 5b shows a moment in which both platforms (2) are inclined by a maximum angle towards one side of the movement. Figure 5c shows a moment in which both platforms (2) are inclined by the same maximum angle towards the opposite side of the movement.

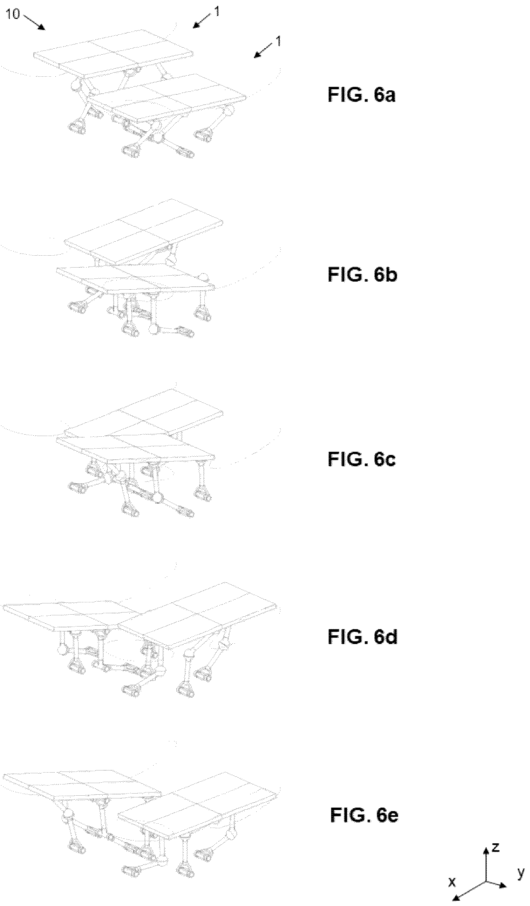

Figures 6a-6c show a third example of a sequence of movements that the aforementioned installation (10) can perform. In this third example, the platforms (2) undergo a combination of displacements along the X and Z axes combined with rotations about axes parallel to the Y axis that simulate the walking motion. It is a sequence of movements similar to those carried out by the well-known exercise machines known as "elliptical trainers". This name is due to the fact that each of the points on each platform (2) follows an elliptical path contained in the corresponding vertical plane. For example, Figures 6a-6c graphically show the path travelled by two corners of each platform (2). If the platform (2) located in the foreground is taken into account, the path followed by the rear left corner thereof (located on the right in the figures) is clear, being an ellipse contained in the second plane (π₃₂) of that device (1).

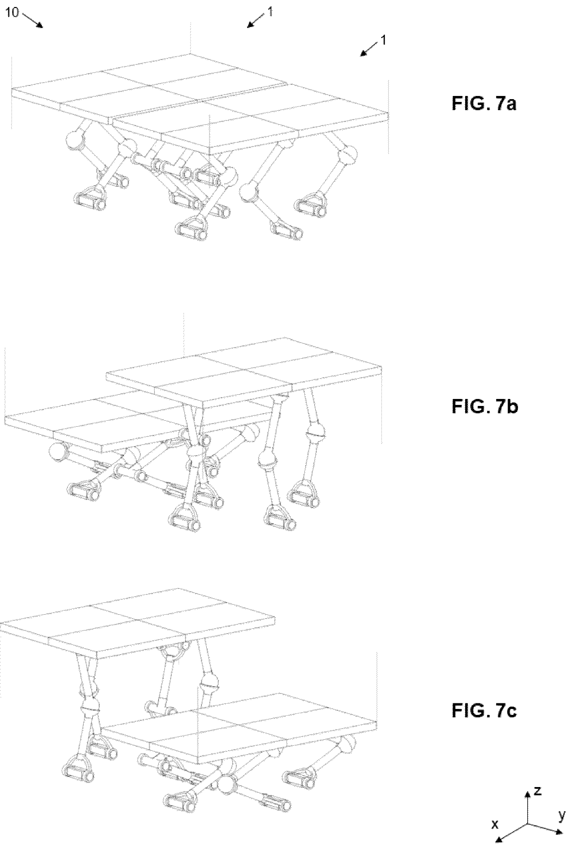

Figures 7a-7c show a fourth example of a sequence of movements that the aforementioned installation (10) can perform. In this fourth example, the platforms (2) move alternately upwards and downwards in the direction of the Z axis, such that while one is moving upwards, the other is moving downwards, both arriving at the same time at the respective change of direction points. Therefore, it is a pure vertical translation movement. Figure 7a shows a moment in which a platform (2) that moves upwards and a platform (2) that moves downwards intersect at an intermediate point in their paths. Figure 7b shows both platforms (2) at their change of direction points, that is, the platform (2) located in the foreground has reached its highest point and the platform (2) located in the background has reached its lowest point. Figure 7c shows the opposite situation, with the platform (2) located in the foreground at its lowest point and the platform (2) located in the background at its highest point.

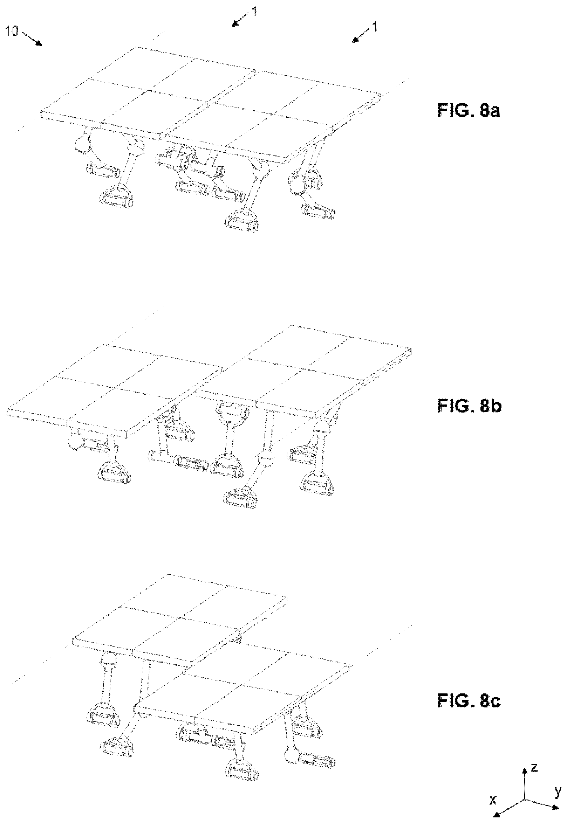

Figures 8a-8c show a fifth example of a sequence of movements that the aforementioned installation (10) can perform. In this fifth example, the platforms (2) move alternately forwards and backwards in the direction of the X axis, such that while one is moving forwards, the other is moving backwards, both arriving at the same time at the respective change of direction points. During the entire movement, both platforms (2) are at the same height, that is, they are contained in the same XY plane. Therefore, it is a pure horizontal translation movement. Figure 8a shows a moment in which a platform (2) that moves forwards and a platform (2) that moves backwards intersect at an intermediate point in their paths. Figure 8b shows both platforms (2) at their change of direction points, that is, the platform (2) located in the foreground has reached its most backward point and the platform (2) located in the background has reached its most forward point. Figure 8c shows the opposite situation, with the platform (2) located in the foreground at its most forward point and the platform (2) located in the background at its most backward point.

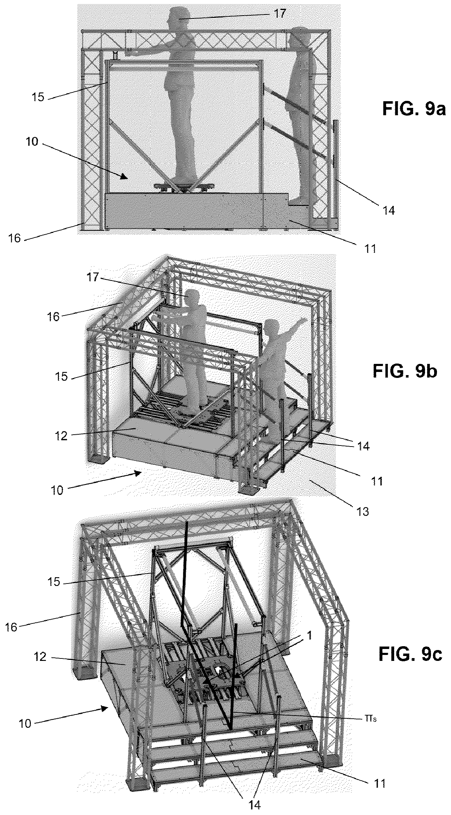

Figures 9a and 9b show a user 17 using the installation 10. The installation 10 comprises steps 11 and an upper surface 12. The upper surface 12 is connected to a lower surface 13 by means of the steps 11. The installation 10 comprises a handrail 14 on each side of the steps.

The upper surface 12 is U-shaped and surrounds the platforms of the device 1. The installation comprises a U-shaped structure 15 that the user 17 can hold directly or, for example, by means of a strap during use of the devices 1.

Figures 9a, 9b and 9c show a structure 16 outside the installation 10. The structure 16 enables components to be coupled that may be useful during the use of the installation 10, such as, for example, a camera to capture images of the user 17, light sources to illuminate the installation 10 or a harness that can be attached to the user 17. The harness improves the stability of the user 17 when using the devices 1.

Figure 9c shows the upper portion of the two devices 1 of the installation 10. The locations of the first, second, third and fourth kinematic chains of one of the devices 1 are symmetrical with the locations of, respectively, the third, second, first and fourth kinematic chains of the other of the devices 1, the symmetry being with respect to the plane πₛ parallel to the X and Z axes. Although not shown, the devices 1 are oriented such that the distance between the fourth kinematic chain of one of the devices of the installation and the fourth kinematic chain of the other of the devices of the installation is the smallest distance between one of the first, second, third and fourth kinematic chains of one of the devices and one of the first, second, third and fourth kinematic chains of the other of the devices (that is, it is the shortest distance between any of said kinematic chains).

## Claims

1. A device (1) for diagnosing and rehabilitating balance that comprises a platform (2) supported by a kinematic mechanism (3) configured to impart rotation movements, translation movements, and combined rotation and translation movements to the platform (2),
**characterised in that** the kinematic mechanism (3) comprises first, second and third kinematic chains (31, 32, 33) that are the same as each other and a fourth kinematic chain (34), wherein:
- each of the first, second and third kinematic chains (31, 32, 33) comprises a proximal rod (BP₃₁, BP₃₂, BP₃₃) attached to a distal rod (BD₃₁, BD₃₂, BD₃₃),
wherein each proximal rod (BP₃₁, BP₃₂, BP₃₃) is contained in a corresponding plane (π₃₁, π₃₂, π₃₃) parallel to a Z direction and an X direction, wherein said X direction and a Y direction perpendicular thereto are contained in an XY base plane that is perpendicular to said Z direction,
wherein each proximal rod (BP₃₁, BP₃₂, BP₃₃) comprises an actuated proximal rotational couple (PRPA₃₁, PRPA₃₂, PRPA₃₃) about an axis (EPRP₃₁, EPRP₃₂, EPRP₃₃) parallel to the Y direction at a proximal end configured for attachment to a fixed base (B), and a passive intermediate spherical couple (PEI₃₁, PEI₃₂, PEI₃₃) at an intermediate end attached to the distal rod (BD₃₁, BD₃₂, BD₃₃), and
wherein each distal rod (BD₃₁, BD₃₂, BD₃₃) comprises a passive distal spherical couple (PED₃₁, PED₃₂, PED₃₃) at the distal end thereof attached to a proximal face (2p) of the platform (2); and
- the fourth kinematic chain (34) comprises a proximal rod (BP₃₄) attached to a distal rod (BD₃₄), the fourth kinematic chain (34) being contained in a plane (π₃₄) parallel to the planes (π₃₁, π₃₂, π₃₃),
wherein the proximal rod (BP₃₄) comprises an actuated proximal rotational couple (PRPA₃₄) about an axis (E_{PRP34}) parallel to the Y direction at a proximal end configured for attachment to the fixed base, and a passive intermediate rotational couple (PRI₃₄) about an axis (E_{PRI34}) parallel to the Y direction at an intermediate end attached to the distal rod (BD₃₄), and
wherein the distal rod (BD₃₄) comprises a universal joint (JU₃₄) which has a first axis (E_{JU34}) parallel to the Y direction at a distal end attached to the proximal face (2p) of the platform (2).

2. The device (1) according to claim 1, wherein a first plane (π₃₁) containing the proximal rod (BP₃₁) of the first kinematic chain (31) and a third plane (π₃₃) containing the proximal rod (BP₃₃) of the third kinematic chain (33) coincide.

3. The device (1) according to claim 2, wherein a second plane (π₃₂) containing the proximal rod (BP₃₂) of the second kinematic chain (32) and a fourth plane (π₃₄) containing the fourth kinematic chain (34) are equidistant in relation to the plane (π₃₁, π₃₃) containing the first and third proximal rods (BP₃₁, BP₃₃) of the first and third kinematic chains (31, 33), and said second and fourth planes (π₃₂, π₃₄) are located on opposite sides in relation to said plane (π₃₁, π₃₃).

4. The device (1) according to claim 3, wherein a distance between the first passive distal spherical couple (PED₃₁) and an attachment line between the universal joint (JU₃₄) and the second passive distal spherical couple (PED₃₂) is the same as a distance between the third passive distal spherical couple (PED₃₃) and said attachment line between the universal joint (JU₃₄) and the second passive distal spherical couple (PED₃₂).

5. The device (1) according to any of claims 3 to 4, wherein a distance between the second passive distal spherical couple (PED₃₂) and an attachment line between the first passive distal spherical couple (PED₃₁) and the third passive distal spherical couple (PED₃₃) is the same as a distance between the universal joint (JU₃₄) and said attachment line between the first passive distal spherical couple (PED₃₁) and the third passive distal spherical couple (PED₃₃).

6. The device (1) according to any of the preceding claims, wherein a distal face (2d) of the platform (2) comprises sensors configured to determine position and reaction force when a patient's foot rests on said distal face (2d) of the platform (2).

7. An installation (10) for diagnosing and rehabilitating balance that comprises two devices (1) according to any one of the preceding claims, wherein said devices (1) are located next to each other according to the Y direction.

8. The installation (10) according to claim 7, the locations of the first, second, third and fourth kinematic chains (31, 32, 33, 34) of one of the devices (1) of the installation (10) being symmetrical to the locations of, respectively, the third, second, first and fourth kinematic chains of the other device of the installation (10), the symmetry being with respect to a plane (πₛ) parallel to the X and Z axes located between the two devices (1) of the installation (10); the distance between the fourth kinematic chain of one of the devices of the installation (10) and the fourth kinematic chain of the other of the devices (1) of the installation (10) being the smallest distance between one of the first, second, third and fourth kinematic chains of one of the devices (1) and one of the first, second, third and fourth kinematic chains of the other of the devices (1).

9. The installation (10) according to any one of claims 7 to 8, the installation (10) comprising steps (11) and/or a ramp, the steps and/or the ramp connecting a lower surface (13) according to the Z axis with an upper surface (12) according to the Z axis, the upper surface (12) being on the fixed bases (B) of the devices (1) of the installation (10), the upper surface (12) being a surface to access the devices (1).

10. The installation (10) according to claim 9, the upper surface (12) being below, according to the Z axis, the platforms of the devices (1) of the installation (10).

11. An operating method of an installation (1) according to any one of claims 7 to 10, which comprises actuating the respective kinematic mechanisms (3) of the two devices (1) to impart a coordinated movement to the platforms (2).

12. The operating method according to claim 11, which comprises actuating the kinematic mechanisms (3) of the two devices (1) to impart a rotation movement to the platforms (2) about an axis parallel to the X direction while maintaining the distal faces (2d) of both platforms (2) contained in the same plane.

13. The operating method according to claim 11, which comprises actuating the kinematic mechanisms (3) of the two devices (1) to impart a rotation movement to the platforms (2) about an axis parallel to the Y direction while maintaining the distal faces (2d) of both platforms (2) contained in the same plane.

14. The operating method according to claim 11, which comprises actuating the kinematic mechanisms (3) of the two devices (1) to impart alternate rotation movements to the platforms (2) about an axis parallel to the second direction (Y) combined with translation movements according to directions parallel to the X direction and the Z direction that simulate the walking motion.

15. The operating method according to claim 11, which comprises actuating the kinematic mechanisms (3) of the two devices (1) to impart an alternate translation movement to the platforms (2) in a direction parallel to the Z direction.

16. The operating method according to claim 11, which comprises actuating the kinematic mechanisms (3) of the two devices (1) to impart an alternate translation movement to the platforms (2) in a direction parallel to the X direction.
